# FASCICULE DE BREVET EUROPEEN

(11) **EP 2 142 270 B1**
(45) Date de publication et mention de la délivrance du brevet: **20.07.2011**
(21) Numéro de dépôt: 08736463.4
(22) Date de dépôt: 22.04.2008
(51) Int. Cl.: B01D 11/04, B01D 11/02, C12P 23/00, C12P 1/00, C12R 1/89, C12M 1/00

(54) **Procédé d'extraction intensif de composés cellulaires issus de microorganismes, par mise en culture et extraction continues, et dispositif correspondant**
Verfahren zur intensiven Extraktion von zellulären Verbindungen aus Mikroorganismen durch kontinuierliche Kultur und Extraktion, und entsprechende Vorrichtung
Method for the intensive extraction of cellular compounds from micro-organisms by continuous culture and extraction, and corresponding device

(30) Priorité: 27.04.2007 FR 0703070
(43) Date de publication de la demande: 13.01.2010
(73) Titulaire: Université de Nantes, 44035 Nantes Cedex 1 (FR); Centre National de la Recherche Scientifique - CNRS, 75794 Paris Cedex 16 (FR)
(72) Inventeur: PRUVOST, Jérémy, F-44250 Saint Brevin (FR); LEGRAND, Jack, F-44600 Saint Nazaire (FR); FOUCAULT, Alain, F-44600 Saint Nazaire (FR)
(74) Mandataire: Larcher, Dominique
(86) Numéro de dépôt international: PCT/EP2008/054869
(87) Numéro de publication internationale: WO 2008/135382

(56) Documents cités:
- WO-A-03/092853
- WO-A-03/095397
- US-A- 5 110 319
- SUTHERLAND ET AL: "Recent progress on the industrial scale-up of counter-current chromatography" JOURNAL OF CHROMATOGRAPHY A, ELSEVIER, AMSTERDAM, NL, [Online] vol. 1151, no. 1-2, 1 mars 2007 (2007-03-01), pages 6-13, XP022063764 ISSN: 0021-9673 Extrait de l'Internet: URL:http://www.sciencedirect.com> [extrait le 2007-11-09]
- MARCHAL L ET AL: "Centrifugal partition chromatography: a survey of its history, and our recent advances in the field" CHEMICAL RECORD, JOHN WILEY, NEW YORK, NY, US, vol. 3, no. 3, 2003, pages 133-143, XP002452497 ISSN: 1527-8999
- DATABASE WPI Week 198824 Thomson Scientific, London, GB; AN 1988-166137 XP002499678 & JP 63 105683 A (SHOKUHIN SANGYO BIOREACTOR) 10 mai 1988 (1988-05-10)

## Description

Le domaine de l'invention est celui des procédés d'extraction de composés cellulaires.

Différentes applications de l'invention peuvent être envisagées, et notamment l'extraction de :
- molécules bioactives (pigments, vitamines...), pour les domaines de la pharmacie, la cosmétique et l'agro-alimentaire ;
- molécules à haut pouvoir énergétique (lipides) pour le domaine de l'énergie.

Plus précisément, l'invention concerne un procédé et un dispositif d'extraction intensif et biocompatible de composés intracellulaires issus de microorganismes photosynthétiques.

L'un des intérêts des microorganismes photosynthétiques tels que les microalgues et cyanobactéries réside dans leur composition originale.

La plupart de ces composés implique toutefois d'appliquer des conditions de stress à la culture afin de jouer sur la flexibilité métabolique importante de ce type de microorganismes pour mener à bien la biosynthèse du composé voulu.

Lorsque ce composé est intracellulaire (comme les pigments notamment), il est nécessaire de mettre en place une seconde étape d'extraction qui, dans la majeure partie des cas, provoque des dommages irréversibles à la culture (broyage choc thermique ou osmotique, désintégration cellulaire...).

Les productions industrielles basées sur ce principe ont donc recours à des productions discontinues, alternant successivement phases de production de biomasse, de mise en conditions de stress, puis phases de récoltes, extraction et purification.

L'inconvénient majeur des productions discontinues est lié à la faible vitesse de croissance des microorganismes photosynthétiques (en comparaison des microorganismes hétérotrophes tels que les bactéries ou les levures), qui empêche des récoltes fréquentes, ou tout du moins impose de travailler avec plusieurs systèmes de production en parallèle.

Pour certains composés, il est possible de mettre en place une technique d'extraction originale, ou seul le composé est extrait, sans altération importante de la cellule (extraction biocompatible). Ceci permet de produire en continu le composé, tout en évitant la répétition de phase de croissance et de stress.

En effet, si cette technique est associée à une production en continu de biomasse en photobioréacteur, une fois la phase initiale de croissance réalisée, les conditions de stress appliquées ensuite peuvent être maintenues en théorie indéfiniment dans la mesure où le composé alors produit est continuellement extrait.

Il en résulte un gain de productivité non négligeable par rapport aux procédés discontinus, les pertes liées au temps de latence nécessaire avant d'obtenir à nouveau une biomasse présentant la composition cellulaire désirée étant évitées.

Une telle technique de production-extraction continues a été proposée par Hejazi et Wijffels en 2003 (**document de brevet publié sous le numéro** EP-1 501 937) et est décrite en référence à la figure 1.

Ce dispositif repose également sur les travaux ayant mis en évidence l'existence de solvants biocompatibles permettant l'extraction de composés valorisables, en l'occurrence du β-carotène issu de la microalgue *Dunaliella salina* (travaux de Leon et al., 2003).

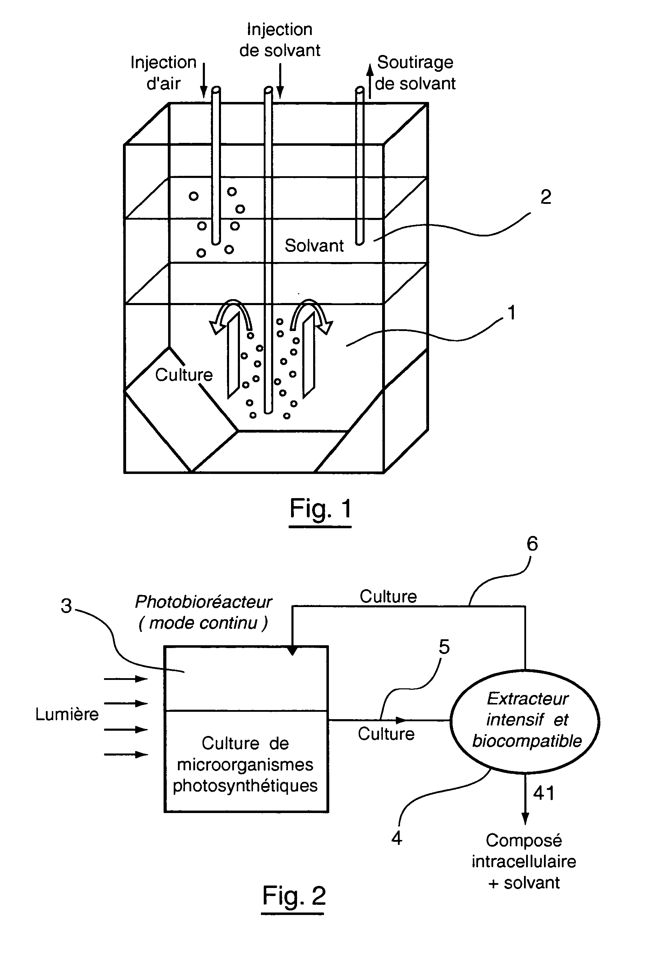

Tel qu'illustré par la figure 1, ce dispositif comprend un photobioréacteur où coexistent la culture 1 contenant le composé à extraire et le solvant 2 où ce composé est progressivement extrait (système biphasique).

Bien qu'opérationnel, ce procédé montre différentes limitations. En effet, la vitesse d'extraction reste relativement faible, du fait de la difficulté de mettre en contact les deux phases non miscibles (cultures en milieu aqueux, et solvant hydrophobe). L'interface de contact restant faible, il en résulte une importante limitation du transfert de matière. La solution consiste à mélanger très fortement l'ensemble, ce qui atteint toutefois rapidement une limite donnée par la fragilité des cellules cultivées.

Un autre inconvénient réside dans l'impossibilité d'imposer des conditions optimales, les deux processus (biosynthèse et extraction) étant différents. A titre d'exemple, le β-carotène extrait étant photosensible, l'utilisation d'un fort éclairement (condition de stress nécessaire à la biosynthèse) pour la culture impose de soutirer régulièrement le solvant chargé en pigment avant dégradation.

Le brevet US5110319 divulgue un procédé d'extraction d'éthanol à partir de moûts de fermentation en utilisant un solvant biocompatible, dans lequel le moût de fermentation est extrait dans une colonne d'extraction en contre-courant avec le solvant d'extraction. Le moût extrait est alors recyclé vers le fermenteur.

L'invention a notamment pour objectif de pallier les inconvénients de l'art antérieur.

Plus précisément, l'invention a pour objectif de proposer une technique de production-extraction continue de composés cellulaires issus de microorganismes permettant d'envisager des vitesses de production et d'extraction augmentées par rapport aux techniques de l'art antérieur.

L'invention a aussi pour objectif de fournir une telle technique qui entraîne l'application de conditions opératoires particulières adaptées aux composés extraits, soit pour en protéger les propriétés après extraction, soit pour améliorer la pureté des composés extraits, par choix de solvant sélectif et maîtrise du temps d'extraction.

Un autre objectif de l'invention est de fournir une telle technique qui permette l'extraction tant de composés intracellulaires que de composés extracellulaires. Ces objectifs, ainsi que d'autres qui apparaîtront par la suite, sont atteints grâce à l'invention qui a pour objet un procédé d'extraction de composés cellulaires issus de micro-organismes, du type comprenant :
- une étape de mise en culture desdits microorganismes ;
- une étape d'extraction desdits composés cellulaires issus desdits micro-organismes,
caractérisé en ce que chaque étape est réalisée en continu, ladite étape d'extraction étant réalisée de façon séparée de ladite étape de mise en culture, ladite étape d'extraction étant réalisée par chromatographies de partage centrifuge et dans des conditions de biocompatibilité avec lesdits micro-organismes à l'aide d'un solvant fortement apolaire, tel que le décane ou le dodécane, étant suivie :
- d'au moins une étape de récupération desdits composés cellulaires ;
- d'au moins une étape de recirculation desdits microorganismes vers ladite étape de mise en culture.

Dans la mesure où le procédé repose sur deux étapes distinctes et continue de mise en culture et d'extraction de composés cellulaires, l'invention s'applique tant à des microorganismes photosynthétiques qu'à des microorganismes non photosynthétiques (bactéries, levures). Seule l'étape de mise en culture diffère, le photobioréacteur (dans le cas de microorganismes photosynthétiques) pouvant être remplacé par tout autre bioréacteur en adéquation avec le microorganisme utilisé.

Chacune des phases (production par mise en culture et extraction) reposant sur des principes, technologies et paramètres très différents, le fonctionnement en deux sous-systèmes selon l'invention permet d'avoir un procédé global intensifié avec possibilité d'un contrôle poussé et indépendant de chaque sous-système.

Il faut noter que l'invention s'applique de façon privilégiée à l'extraction d'un composé intracellulaire. Toutefois, dans certains cas, il est possible que les microorganismes relarguent dans le milieu un métabolite (métabolite extracellulaire). L'invention s'applique également à ce cas, avec le même principe global de fonctionnement, la seule différence étant que le métabolite est alors extrait du milieu de culture par l'extracteur (extraction liquide-liquide), au lieu de la cellule directement (extraction solide-liquide).

Que le métabolite soit intra ou extra-cellulaire, le concept de fonctionnement en boucle des deux sous-systèmes n'est toutefois possible que si la phase d'extraction est non destructive vis à vis du matériel biologique, afin que les cellules vivantes soient réinjectées dans le sous-système de production.

Si ce n'est pas le cas, les deux sous-systèmes fonctionnent en cascade, ce qui est un schéma classique au niveau industriel. L'étape d'extraction doit donc éviter les dommages irréversibles à la culture, qu'ils soient mécaniques, thermiques, ou chimiques. Un choix adéquat du système d'extraction permet d'éviter les dommages mécaniques et les chocs thermiques.

L'originalité de l'invention est donc la division en deux sous-systèmes optimisés, fonctionnant de façon couplée, chacun répondant aux contraintes permettant finalement la production-extraction continue. Par rapport au système existant, deux intérêts majeurs sont apportés :
- possibilité d'accroissement des transferts de matière au niveau de l'extracteur (et donc meilleur rendement global) ;
- possibilité d'appliquer des conditions d'extraction différentes de celles de production pour préserver l'intégrité du composé intracellulaire extrait.

En résumé, l'invention permet des améliorations majeures tant en ce qui concerne le transfert de matière, qu'en ce qui concerne la possibilité offerte d'appliquer des conditions optimales opératoires pour les deux étapes (production et extraction), ceci par la mise en place de deux sous-systèmes dédiés à chaque étape et fonctionnant de façon couplée, à savoir avec une production en continu et sous condition de stress du composé intracellulaire, et une partie extraction biocompatible de ce composé.

On note que, même si a priori la complexité du procédé est accrue par l'utilisation des deux sous-systèmes, le couplage est facilité par le fonctionnement en régime continu et permanent. Il est ainsi possible d'optimiser les conditions de culture pour améliorer l'extraction (le maintien d'une concentration intracellulaire constante dans le temps permet de mieux définir les conditions d'extraction à appliquer).

Selon une solution avantageuse, ladite étape de mise en culture est une étape de bioproduction, apportant au milieu vivant les conditions nécessaires pour la synthèse du métabolite recherché.

Avantageusement, ladite étape d'extraction est une étape d'extraction liquide-liquide (métabolite extracullaire) ou solide-liquide (métabolite intracellulaire).

Un tel mode d'extraction permet d'assurer le transfert du métabolite recherché de la cellule (cas d'un composé intracellulaire) et/ou du milieu de culture (cas d'un composé extracellulaire exudé) à un solvant récupéré ensuite en sortie de l'extracteur.

L'étape d'extraction est réalisée par Chromatographie de Partage Centrifuge (CPC).

Ainsi, les fortes capacités de transfert de la CPC permettent de disposer d'un système d'extraction liquide-liquide haute performance.

L'utilisation de la CPC implique toutefois une contrainte majeure, à savoir la possibilité de fortes sollicitations mécaniques subies par les cellules vivantes lors de leur traversée de l'appareil (champ centrifuge, système de pompage de la culture, cisaillement entre et dans chaque cellule de séparation). Le système n'est donc utilisable en continu que si la culture est capable de supporter ce traitement. Des essais ont montré la faisabilité de l'approche et son potentiel (rendements accrus par rapport à la littérature), à condition toutefois de rester dans une gamme opératoire de la CPC acceptable pour préserver l'intégrité cellulaire du matériel biologique.

Selon une variante envisageable, ladite étape d'extraction est réalisée sous obscurité.

Selon une autre variante envisageable, ladite étape d'extraction est réalisée en anoxie sous azote gazeux.

On comprend donc qu'un procédé selon l'invention permet d'appliquer facilement des conditions particulières dans le système d'extraction, comme le travail à l'obscurité si le produit est photosensible, ou en conditions d'anoxie, si le produit s'oxyde rapidement. De même, le mode d'extraction retenu peut être choisi pour amener une grande sélectivité (choix du solvant et maîtrise du temps d'extraction).

L'invention concerne également un dispositif pour la mise en oeuvre d'un procédé tel que décrit précédemment, comprenant :
- au moins une cuve de mis en culture desdits microorganismes,
- au moins une cuve d'extraction fortement apolaire, tel que le décane ou le dodécane, desdits composés cellulaires issus desdits microorganismes, ladite cuve d'extraction étant distincte de ladite cuve de mise en culture et contenant au moins un solvane biocompatible avec lesdits microorganismes ;
- des moyens de récupération desdits composés cellulaires ;
- des moyens de recirculation desdits microorganismes à partir de ladite cuve d'extraction dans ladite cuve de mise en culture.

On note que l'invention évite aux microorganismes les dommages d'origines chimiques, ceci par la mise en oeuvre d'un solvant biocompatible, qui extrait le métabolite intracellulaire, sans altération irréversible du métabolisme du microorganisme pouvant mener à la mort cellulaire.

Selon une solution avantageuse, ladite cuve de mise en culture est un photobioréacteur, et ladite cuve d'extraction est une cuve d'extraction liquide-liquide.

D'autres caractéristiques et avantages de l'invention apparaîtront plus clairement à la lecture de la description suivante d'un mode de réalisation préférentiel de l'invention, donné à titre d'exemple illustratif et non limitatif, et des dessins annexés parmi lesquels :
- la figure 1 est une représentation schématique d'une technique de production-extraction inventive selon l'art antérieur ;
- la figure 2 est une représentation schématique d'un dispositif pour la mise en oeuvre d'un procédé selon l'invention ;
- la figure 3 est un graphique montrant des résultats d'extraction par CPC du β-carotène intracellulaire d'une culture de Dunaliella salina, à l'aide d'un procédé selon l'invention.

Tel qu'indiqué précédemment, le principe de l'invention réside dans le fait de proposer un procédé de production-extraction des composés cellulaires reposant sur l'association de deux sous-systèmes fonctionnant en boucle, chacun étant dédié spécifiquement à une des deux phases de production et d'extraction.

Un dispositif pour la mise en oeuvre d'un procédé selon l'invention est décrit en référence à la figure 2.

Un tel dispositif comprend :
- un photobioréacteur 3 fonctionnant en mode continu assurant l'étape de mise en culture des microorganismes photosynthétiques ;
- un extracteur 4 assurant en continu l'extraction des composés cellulaire
- des moyens de transfert 5 des microorganismes du photobioréacteur dans l'extracteur 4 ;
- des moyens de récupération 41 des composés cellulaires ;
- des moyens de recirculation 6 des microorganismes de l'extracteur 4 dans le photobioréacteur 3.

La phase de biosynthèse est ainsi réalisée dans un ou plusieurs photobioréacteurs fonctionnant en continu, permettant d'appliquer des conditions idéales et maîtrisées pour mener à une vitesse de biosynthèse optimisée du composé désiré. En modifiant les paramètres physico-chimiques de culture (température, pH, milieu de culture, lumière incidente), il est ainsi possible de favoriser par induction d'un stress physiologique la biosynthèse d'un métabolite spécifique, intervenant par exemple dans la protection du microorganisme à ce stress (exemple du β-carotène en situation de stress lumineux et oxydatif)

L'extracteur est constitué d'une cuve contenant un solvant biocompatible tel que le dodécane ou le décane.

Avec un tel solvant, on procède à une extraction biocompatible. Il est alors possible d'extraire en continu et de façon sélective certains composés intracellulaires tout en préservant la vitalité des cellules.

Dans le cas d'un solvant faiblement hydrophobe, la mise en contact altère de façon irrémédiable la structure cellulaire. L'extraction est alors très importante, mais non sélective (une grande quantité de composants sont extraits) et non biocompatible.

Par contre, si le solvant est choisi suffisamment apolaire, il est possible de préserver la paroi cellulaire et les fonctions vitales, tout en maintenant une extraction de certains composants hydrophobes, et notamment la chlorophylle et le β-carotène chez *Dunaliella salina.* Plus le solvant est apolaire et plus la capacité d'extraction diminue, avec une réduction plus marquée pour la chlorophylle. En choisissant un solvant adéquat, une extraction biocompatible et sélective du β-carotène peut donc être obtenue.

En comparaison des méthodes brutales d'extraction conventionnelles mais quasi-complètes des pigments intracellulaires, la perte de productivité générée par l'utilisation d'un solvant fortement apolaire a priori moins efficace peut alors être compensée par le maintien d'une extraction continue de β-carotène directement issu de cellules en culture.

Selon le principe de l'invention, le solvant est maintenu dans un système parallèle dédié à l'extraction (l'extracteur 4, clairement séparé physiquement du photobioréacteur 3) dans lequel transite la culture avant retour dans le(s) photobioréacteur(s) de production.

Avec un tel dispositif, et en appliquant une importante percolation par exemple (la phase dispersée est la culture, et la phase continue le solvant), il en résulte une augmentation importante des surfaces interfaciales entre le milieu biologique aqueux et le solvant (phases non miscibles). Même si cela génère des sollicitations mécaniques élevées (stress hydrodynamiques), ceci est compensé par un temps de passage court dans le sous-système d'extraction, compte tenu de la vitesse élevée d'extraction.

L'étape d'extraction est réalisée par Chromatographie de Partage Centrifuge (CPC) : comparable à la CLHP (Chromatographie Liquide Haute Performance), cette technique en diffère cependant par l'absence de support solide et son importante capacité de traitement.

Dans la technique CPC, la partie utile est constituée d'un disque en rotation munie d'une série de cellules de séparation. Grâce à un important champ de forces centrifuges, l'une des phases, appelée stationnaire, est maintenue dans l'appareil. L'autre phase (mobile) circulant avec un débit forcé percole alors au niveau de chaque cellule dans la phase stationnaire, d'où une amélioration significative de la mise en contact des deux phases liquides. L'efficacité dépend alors des conditions opératoires (vitesse de rotation, débit de la phase mobile), des propriétés physiques des deux phases et de la forme de chaque cellule de séparation (Marchal 2001). Cette méthode est appliquée usuellement à des purifications de molécules issues de mélanges complexes : composés naturels actifs, peptides, acides gras, phospholipides, antibiotiques, extraction d'arômes, fractionnement de produits pétroliers, ...

Suivant l'application visée, on envisagera l'utilisation de photobioréacteurs de grands volumes, de bassins de culture, ou de photobioréacteurs de plus faibles volumes mais intensifiés. L'extracteur sera un extracteur intensifié CPC, dans lequel on optimise les transferts de matière et la mise en contact du milieu de culture avec le solvant extracteur.

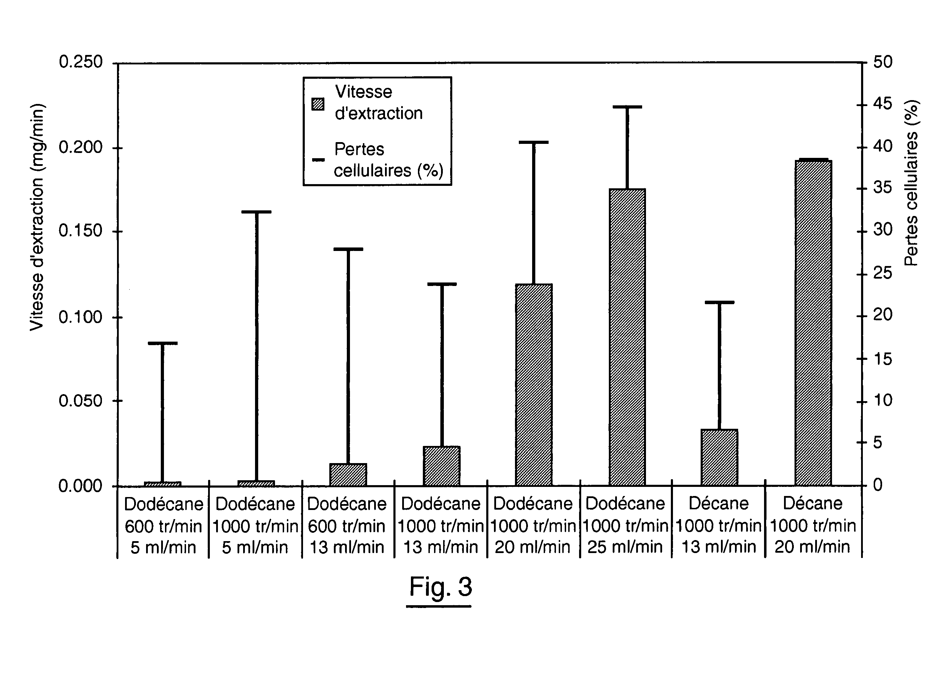

Pour montrer la faisabilité du couplage CPC-photobioréacteur, des essais ont été réalisés et ont notamment visé à évaluer la quantité de β-carotène extraite et les pertes cellulaires associées, ceci en fonction de divers paramètres opératoires de la CPC, en utilisant le dodécane et le décane comme solvants. Quelques exemples de résultats sont présentés par la figure 3.

Ces résultats mettent en valeur trois des principales caractéristiques du procédé, à savoir :
- le rôle primordial du transfert de matière lors de l'extraction ;
- le besoin de trouver un compromis pour respecter l'intégrité cellulaire et ;
- l'influence de la solubilité du β-carotène dans le solvant.

Le débit d'alimentation en phase mobile (culture) dans la CPC joue ainsi un rôle important. Une quantité plus élevée de β-carotène est extraite à fort débit, mais avec une altération conséquente des cellules vivantes (perte de flagelles, mortalité).

Du fait de la capacité du milieu vivant à se régénérer, une faible perte de biomasse est toutefois acceptable. Ainsi, 48h après passage dans la CPC sous des conditions sévères, une grande partie de la culture est recouvrée (pertes par rapport à la concentration initiale de 40% à 20%).

Ceci montre la diversité des protocoles opératoires possibles, avec soit une extraction modérée mais permanente, soit une extraction importante mais séquentielle, avec des temps de latence à définir pour régénérer le matériel vivant entre chaque extraction.

On notera que ces différentes possibilités de fonctionnement ne modifient en rien et sont englobées dans le principe global de l'invention décrit préalablement (fonctionnement en deux sous-systèmes).

On rappelle que la séparation des deux étapes de production et d'extraction permet d'optimiser chacune des phases (et leur couplage).

Concernant la partie production, il est important de trouver les conditions permettant de maximiser la composition intracellulaire en β-carotène. Ces conditions doivent être maintenues en régime continu. Les conditions d'utilisation de la CPC peuvent alors plus facilement être optimisées.

Du fait de la rupture apportée par le fonctionnement en deux sous-systèmes, différents essais ont été réalisés, afin de mettre en place des nouveaux protocoles spécifiques.

En effet, la plupart des protocoles existant ont été développés sur la base d'une culture extensive et discontinue.

Le fonctionnement en photobioréacteur continu apporte un nouveau point de vue à optimiser.

Ainsi, la concentration intracellulaire usuellement aux alentours de 15-20 pg par cellules a été augmentée à 70.

Ceci a été obtenu par forçage physiologique en jouant sur les conditions opératoires classiques de culture en photobioréacteur (température, irradiance, pH) et en modifiant le milieu de culture. Deux composés ont ainsi été ajoutés au milieu de culture standard, à savoir de l'acétate et du fer (Fe²⁺), ces deux composés permettant de favoriser à la fois la croissance et la biosynthèse du β-carotène. Il faut noter que le fonctionnement en culture continue permet d'optimiser aisément la quantité respective de chacun des composés (quantité par cellule constante dans le temps).

L'association de la CPC à un photobioréacteur rectangulaire simple de 4 litres et 3cm de chemin optique fonctionnant en continu où ont été appliquées les conditions menant à la biosynthèse du β-carotène a permis d'obtenir des premiers résultats qui confortent les différentes améliorations apportées par chaque sous-système. Ainsi, les études de l'art antérieur menées sur l'extraction du β-carotène en réacteur simple ont montré que 5 à 10 mg/l pouvaient être extraits au maximum.

La CPC a permis d'obtenir une extraction de 148 mg/l de β-carotène en tête de colonne, ce qui est 30 fois plus que les méthodes utilisées selon l'art antérieur (conditions d'extraction par CPC ; débit d'alimentation 20ml/mn - Vitesse de rotation 1000 tr/min - Solvant Décane ; Conditions de culture : Photobioréacteur rectangulaire de 4 litres - ph 7,5 - Température 30°C - Salinité 220g/l - Lumière incidente 700 µE/m².s). Au total, 1,25 mg de β-carotène ont été extraits pour 3200 ml de culture et 60 ml de solvant.

En production continue, une productivité de l'ordre de 50mg de caroténoïdes par jour et litre de culture est attendue.

## Revendications

1. Procédé d'extraction de composés cellulaires issus de micro-organismes, du type comprenant :
- une étape de mise en culture desdits microorganismes ;
- une étape d'extraction desdits composés cellulaires issus desdits micro-organismes,
**caractérisé en ce que** chaque étape est réalisée en continu, ladite étape d'extraction étant réalisée de façon séparée de ladite étape de mise en culture, ladite étape d'extraction étant réalisée par chromatographie de partage centrifuge, et dans des conditions de biocompatibilité avec lesdits micro-organismes à l'aide d'un solvant fortement apolaire, tel que le décane ou le dodécane, et étant suivie :
- d'au moins une étape de récupération desdits composés cellulaires ;
- d'au moins une étape de recirculation desdits microorganismes vers ladite étape de mise en culture.

2. Procédé d'extraction selon la revendication 1, **caractérisé en ce que** ladite étape de mise en culture est une étape de production biologique.

3. Procédé d'extraction selon l'une des revendications 1 et 2, **caractérisé en ce que** ladite étape d'extraction est une étape d'extraction liquide-liquide ou solide-liquide.

4. Procédé d'extraction selon l'une quelconque des revendication 1 à 3, **caractérisé en ce que** ladite étape d'extraction est réalisée sous obscurité.

5. Procédé d'extraction selon l'une quelconque des revendication 1 à 3, **caractérisé en ce que** ladite étape d'extraction est réalisée en anoxie sous azote gazeux.

6. Dispositif pour la mise en oeuvre du procédé d'extraction selon l'une quelconque des revendications 1 à 5, **caractérisé en ce qu'**il comprend :
- au moins une cuve 3 de mise en culture desdits microorganismes ;
- au moins une cuve d'extraction par chromatographie de partage centrifuge 4 desdits composés cellulaires issus desdits microorganismes, ladite cuve d'extraction 4 étant distincte de ladite cuve 3 de mise en culture et contenant au moins un solvant fortement apolaire, tel que le décane ou le dodécane, biocompatible avec lesdits microorganismes ;
- des moyens de récupération 41 desdits composés cellulaires ;
- des moyens de recirculation 6 desdits microorganismes à partir de ladite cuve d'extraction dans ladite cuve de mise en culture.

7. Dispositif selon la revendication 6 **caractérisé en ce que** ladite cuve 3 de mise en culture est un bioréacteur.

8. Dispositif selon des revendications 6 et 7, **caractérisé en ce que** ladite cuve d'extraction 4 est une cuve d'extraction liquide-liquide ou solide-liquide.

## Claims

1. Process for the extraction of cellular compounds derived from microorganisms, of the type comprising:
- a step for the cultivation of said microorganisms;
- a step for the extraction of said cellular
compounds derived from said microorganisms, **characterized in that** each step is carried out continuously, said extraction step being carried out separately from said cultivation step, said extraction step being carried out by centrifugal partition chromatography, under conditions of biocompatibility with said microorganisms, using a strongly apolar solvent such as decane or dodecane, and being followed by:
- at least one step for the recovery of said cellular compounds;
- at least one step for the recycling of said microorganisms to said cultivation step.

2. Extraction process according to Claim 1, **characterized in that** said cultivation step is a biological production step.

3. Extraction process according to Claim 1 or 2, **characterized in that** said extraction step is a liquid-liquid or solid-liquid extraction step.

4. Extraction process according to any one of Claims 1 to 3, **characterized in that** said extraction step is carried out in the dark.

5. Extraction process according to any one of Claims 1 to 3, **characterized in that** said extraction step is carried out anoxically under nitrogen gas.

6. Device for carrying out the extraction process according to any one of Claims 1 to 5, **characterized in that** it comprises:
- at least one tank 3 for the cultivation of said microorganisms;
- at least one tank 4 for the extraction, by centrifugal partition chromatography, of said cellular compounds derived from said microorganisms, said extraction tank 4 being different from said cultivation tank 3 and containing at least one strongly apolar solvent such as decane or dodecane, which is biocompatible with said microorganisms;
- means 41 for the recovery of said cellular compounds;
- means 6 for the recycling of said microorganisms from said extraction tank to said cultivation tank.

7. Device according to Claim 6, **characterized in that** said cultivation tank 3 is a bioreactor.

8. Device according to Claims 6 and 7, **characterized in that** said extraction tank 4 is a liquid-liquid or solid-liquid extraction tank.

## Patentansprüche

1. Verfahren zur Extraktion von zellulären Verbindungen aus Mikroorganismen, des Typs umfassend:
- einen Schritt zum Anlegen einer Kultur dieser Mikroorganismen;
- einen Schritt zur Extraktion der zellulären Verbindungen aus diesen Mikroorganismen,
**dadurch gekennzeichnet, dass** jeder Schritt kontinuierlich ausgeführt wird und der Schritt der Extraktion separat vom Schritt des Anlegens der Kultur ausgeführt wird, wobei der Schritt der Extraktion durch Zentrifugalverteilungschromatographie und unter mit diesen Mikroorganismen biologisch verträglichen Bedingungen mithilfe eines stark unpolaren Lösungsmittels, wie Dekan oder Dodekan, ausgeführt wird, und sich an diesen Schritt anschließt:
- mindestens ein Schritt zur Rückgewinnung dieser zellulären Verbindungen;
- mindestens ein Schritt zur Rückführung der Mikroorganismen zum Schritt des Anlegens der Kultur.

2. Extraktionsverfahren nach Anspruch 1, **dadurch gekennzeichnet, dass** der Schritt des Anlegens der Kultur ein Schritt der biologischen Produktion ist.

3. Extraktionsverfahren nach einem der Ansprüche 1 und 2, **dadurch gekennzeichnet, dass** der Extraktionsschritt ein Schritt der Flüssig-Flüssig-Extraktion oder der Fest-Flüssig-Extraktion ist.

4. Extraktionsverfahren nach einem der Ansprüche 1 bis 3, **dadurch gekennzeichnet, dass** der Extraktionsschritt unter Lichtausschluss ausgeführt wird.

5. Extraktionsverfahren nach einem der Ansprüche 1 bis 3, **dadurch gekennzeichnet, dass** der Extraktionsschritt unter Anoxie unter gasförmigem Stickstoff ausgeführt wird.

6. Vorrichtung zur Durchführung des Extraktionsverfahrens nach einem der Ansprüche 1 bis 5, **dadurch gekennzeichnet, dass** sie umfasst:
- mindestens eine Kammer 3 zum Anlegen einer Kultur der Mikroorganismen;
- mindestens eine Kammer zur Extraktion durch Zentrifugalverteilungschromatographie 4 der zellulären Verbindungen aus diesen Mikroorganismen, wobei die Kammer zur Extraktion 4 von der Kammer 3 zum Anlegen der Kultur verschieden ist und mindestens ein stark unpolares Lösungsmittel, wie Dekan oder Dodekan, das mit diesen Mikroorganismen biologisch verträglich ist, enthält;
- Mittel zur Rückgewinnung 41 der zellulären Verbindungen;
- Mittel zur Rückführung 6 der Mikroorganismen aus der Kammer zur Extraktion in die Kammer zum Anlegen der Kultur.

7. Vorrichtung nach Anspruch 6, **dadurch gekennzeichnet, dass** die Kammer 3 zum Anlegen der Kultur ein Bioreaktor ist.

8. Vorrichtung nach den Ansprüchen 6 und 7, **dadurch gekennzeichnet, dass** die Kammer zur Extraktion 4 eine Kammer zur Flüssig-Flüssig-Extraktion oder zur Fest-Flüssig-Extraktion ist.
